# EUROPEAN PATENT SPECIFICATION

(11) **EP 4 467 167 B1**
(45) Date of publication and mention of the grant of the patent: **18.11.2026**
(21) Application number: 23175524.0
(22) Date of filing: 25.05.2023
(51) Int. Cl.: A61L 27/04, A61L 31/02

(54) **METHOD FOR COATING A MEDICAL DEVICE, ESPECIALLY FOR FABRICATION OF ANTITHROMBOGENIC AND ANTIBACTERIAL TITANIUM MATERIALS AND THEIR ALLOYS**
VERFAHREN ZUR BESCHICHTUNG EINES MEDIZINISCHEN GERÄTS, INSBESONDERE ZUR HERSTELLUNG VON ANTITHROMBOGENEN UND ANTIBAKTERIELLEN TITANWERKSTOFFEN UND DEREN LEGIERUNGEN
PROCÉDÉ DE REVÊTEMENT D'UN DISPOSITIF MÉDICAL, EN PARTICULIER POUR LA FABRICATION DE MATÉRIAUX EN TITANE ANTITHROMBOGÈNES ET ANTIBACTÉRIENS ET LEURS ALLIAGES

(43) Date of publication of application: 27.11.2024
(73) Proprietor: Jozef Stefan Institute, 1000 Ljubljana (SI); Univerza v Ljubljani, 1000 Ljubljana (SI)
(72) Inventor: JUNKAR, Ita, 1000 Ljubljana (SI); BENCINA, Metka, 1000 Ljubljana (SI); RAWAT, Niharika, 1000 Ljubljana (SI); MARKOVIC, Gaja, 1000 Ljubljana (SI); STANIC, Jan, 1000 Ljubljana (SI); KRALJ-IGLIC, Veronika, 1000 Ljubljana (SI); IGLIC, Ales, 1000 Ljubljana (SI)
(74) Representative: Marton, Dan-Robert

(56) References cited:
- EP-A1- 4 049 690
- EP-A1- 4 129 351
- WO-A1-2018/029166
- WO-A1-2022/268035
- US-A- 6 006 763
- SHARMA R ET AL: "Application of Atmospheric-Pressure Plasma for Enhancing Photoelectrochemical Properties of TiO2 Electrodes", INDUSTRY APPLICATIONS SOCIETY ANNUAL MEETING, 2008. IAS '08. IEEE, IEEE, PISCATAWAY, NJ, USA, 5 October 2008 (2008-10-05), pages 1 - 5, XP031353837, ISBN: 978-1-4244-2278-4

## Description

### Field of the invention

The present invention generally relates to a method for the formation (or coating) of micro and nano structured titanium oxide surfaces on titanium (Ti) and Ti alloys.

### Background of the invention

The need for implantable material and medical devices, for instance cardiovascular stents is growing rapidly. This is due to the increased incidence of chronic and cardiovascular diseases (CVDs), ageing of the population and unhealthy style of living. The minimal invasive way to treat diseased blood vessels is by insertion of a metal stent to help restore the blood flow through the affected vessel. Thus, the basic function of a stent is to act as an artificial conduit or substitute for an abnormality in veins or arteries. The life span of implanted medical devices is influenced by many factors such as the materials used, immunological state of the patient, healing process, and the underlying disease itself. The best materials used for implanted devices are most commonly those that best mimic the native vascular tissue. Matching the properties of a native vascular wall completely still remains a challenge and further advancements in the field of biocompatibility are needed.

CVDs are a major reason for the need of an implantable medical device, i.e. cardiovascular stent. Risk factors for developing a CVD include diabetes, smoking, physical inactivity, obesity, hypertension, among others. CVDs are also continuously reported among the leading causes of death globally. The pathology behind CVDs is atherosclerosis, which is a degenerative condition defined as a focal, inflammatory fibre-proliferative response to multiple forms of endothelial injury where we find lesions in the intima of the vascular wall called atheromas. Atherosclerosis is characterized by the formation of plaques with lipid cores and fibrous caps. It is a chronic inflammatory condition with plaques developing over decades. Stable plaques are growing in size and at one point start obstructing the normal blood flow in the arteries which can lead to a number of diseases, clinical complications, and symptoms, such as angina pectoris (chest pain). Atherosclerosis also predisposes a vascular wall to the formation of aneurysms and creates conditions where dissection of an aortic wall is more likely to occur. Both can lead to acute, life treating conditions. A plaque or a thrombus may also break off the vascular wall and create an embolism somewhere in the arterial system. Atherosclerosis is a systemic disease, but some vessels are more likely to be affected or are more likely to result in clinical symptoms than others. This is shown in different clinical manifestations, such as coronary heart disease, carotid artery disease, peripheral artery disease, and renovascular disease among others.

A stent (defined as a tubular metal mesh) is a scaffolding device used to hold tissue in place in a specific stretched or taut position. The procedure that leads to the placing of a stent is called angioplasty and it involves mechanical widening of a narrowed blood vessel with a balloon catheter that forces the expansion of the vessel and the surrounding muscular wall. This in turn improves blood flow and consequently relieves the symptoms of the aforementioned CVDs.

Vascular stents have been predominantly metal structures inserted into partially clogged arteries to promote normal blood flow. Usually, they are made out of hemo-compatible and durable material, such as Titanium (Ti), 316L stainless steel (SS-medical grade), Nitinol (an alloy of Nickel and Titanium), and Cobalt-Chromium (CoCr). Peripheral angioplasty refers to the use of mechanical widening to open blood vessels other than the coronary arteries. This procedure is called percutaneous transluminal angioplasty (PTA), the most commonly used to treat narrowing in the leg arteries. A stent is typically inserted through a main artery in the groin (femoral artery) or arm (brachial artery) on a wire or catheter and extended up to the narrowed section of the vessel.

Stenting procedures are still not optimal. Conventional bare-metal stents (BMS) often trigger restenosis, which is an inflammatory response leading to scar tissue formation and reverses the benefit of placing a stent in the first place and is also one of the major reasons for failure of a stent procedure. Drug-eluting stents (DES) are BMS usually coated with immunosuppressive drugs to prevent restenosis. The DES lowers the risk of restenosis by releasing antiproliferative, immunosuppressive or anti-thrombogenic drugs which inhibit proliferation of smooth muscle cells (SMCs) and reduce thrombus formation. However, these types of stents may cause also suppression of endothelial cell (EC) growth and sometimes can lead to late-stage thrombosis. The other problem encountered with BMS is an allergic reaction, as the release of toxic ions like Ni is still an issue. The risk was significantly reduced by the use of DES. The vascular wall can also be damaged during stenting. As a result, a blood clot forms at the site of injury and reduces blood flow. In addition, an inflammatory response occurs at the site. Afterward, the SMCs are proliferating in the intima because of released growth factors. This is also known as neointimal hyperplasia which causes narrowing in the lumen of a stent. The consequence of these processes is a remodelling of the artery and restenosis. One of the factors contributing to this is also incomplete endothelialization because the drugs delivered are not targeted cell-specific and are preventing the formation of a healthy EC layer, consequently increasing the chance for a thrombogenic event. Due to the high risk of thrombosis, patients should take antiplatelet drugs practically throughout their lives.

In addition, bacterial infections present a serious concern to all implantable materials, as, among all possible complications, implant infections occur in 1% to13% of the cases (Ribeiro M. et al, Biomatter. 2012 2(4):176-94.). This percentage may rise due to the increase in antibiotic-resistant bacteria strains.

Therefore, even the new generation DES is not a perfect solution. Other ways of addressing restenosis and the hyperproliferation of vascular SMCs by promoting rapid reendothelialization over the stent are also sought. There is an inverse relationship between luminal EC coverage and vascular SMC proliferation after arterial injury. New options should be sought, which are not reliant on pharmacological means to fight restenosis through the promotion of reendothelialization and healing.

There is an urgent need to develop multifunctional surfaces of vascular stents, that would at the same time (i) prevent the adhesion, aggregation and activation of platelet; (ii) inhibit the adhesion and proliferation of SMC; (iii) promote the adhesion and proliferation of EC and also (vi) prevent bacterial adhesion and biofilm formation. As nanostructured surfaces were already shown to provide cell guidance and improved proliferation (Higgins, S. et.al, Advanced Materials, 2020, 32(9), 1903862.), it could be possible to design specific surface features to gain selectivity and thus obtain conditions that favour the growth of one cell type over another (EC over SMC). Attempts to fabricate new platforms with additional antibacterial properties and selective proliferation of desired cell type would present a huge step forward in the field of biomedical devices, as till now, surfaces that incorporate all these functionalities still needed to be developed.

Nanomaterials have the potential to provide a solution to the limitations of conventional stents. By modifying the surface topography of the stent material, it is possible to influence the way cells interact with the material. Several studies have shown that nanorough surfaces can promote endothelial cell growth and reduce platelet activation and aggregation. Such surfaces can also limit smooth muscle cell proliferation, which is a major cause of restenosis. Another approach is to use nanocoatings, which can be used to modify the surface topography of a stent. For example, by coating a stent with a layer of nanoparticles, it is possible to create a surface that is anti-thrombogenic and promotes cell growth. Such coatings can also be used to deliver drugs or growth factors directly to the site of the stent implantation, further promoting the healing process. Overall, the use of nanomaterials and nanostructured surfaces in stent design holds great promise for improving the efficacy and safety of these medical devices. Creating surfaces that promote the appropriate biological response can reduce the risk of complications such as restenosis and thrombosis.

A big obstacle in the efficiency and longevity of medical devices is also a bacterial infection in light of growing antimicrobial resistance and biofilm formation. With the control of the diameter in TiO2 nanotubes (amorphous or crystalline) there is a changed response in some of the most common pathogens relevant for medical device-related infections like Staphylococcus epidermidis (S. epidermidis) and Staphylococcus aureus (S. aureus)). The use of larger-diameter nanotubes decreases the number of live bacteria as compared to lower-diameter ones. Also, the crystallinity of the nanotubes influences the cell-specific activity, as annealed nanotubes (to anatase/rutile mixtures) lead to better inhibitory activity toward Pseudomonas aeruginosa and Staphylococcus aureus (Junkar I. Advances in Biomembranes and Lipid Self-Assembly. Elsevier B.V.; 2017. 1). The antibacterial activity of titanium or titanium alloy nanostructures could be due to: the nanostructuring of the implant surface (the diameter size effect), usage of alloys with inherent antimicrobial properties or by decorating the nanostructures with antimicrobial nanoparticles and/or by functionalization of the surface with anti-inflammatory agents or by loading drugs inside the nanostructures.

Nanostructurization of medical devices is therefore a promising area of research that has the potential to impact various fields of medicine. Nanostructured medical devices with improved biocompatibility may reduce adverse effects and improve patient outcomes. Nanostructurization refers to the manipulation of materials at the nanoscale level to create structures that mimic the properties of living tissues. Biocompatibility is an important concern in the development of medical devices since foreign materials introduced into the body can cause immune responses, inflammation, and even rejection. In recent years, several patents have been filed for the development of nanotechnology-based medical devices that improve biocompatibility.

For example, the method described in US7955512 aims to enhance the biocompatibility of medical devices by improving their surface properties. Patent discloses the textured surface (for instance stainless steel and Ti-6Al-4V) accomplished by the use of gas-phase plasma on medical devices with complex geometries, such as stents. The textured surface of the vascular stent is designed to reduce the risk of restenosis (re-narrowing of the artery). By altering the surface morphology at the nano- or micro-scopic level, the stent can promote better endothelialization (formation of a smooth layer of cells lining the inside of the artery) and reduce inflammation and thrombosis (blood clotting). The texturing process using radio-frequency-generated (RF-generated) plasma involves exposing the stent to a low-temperature plasma formed by applying a radio-frequency electric field to a gas in a vacuum chamber. This causes chemical reactions on the surface of the stent, resulting in the formation of textured surfaces on metal substrates. The patent however does not involve the reactions in liquid phase nor it uses atmospheric pressure plasma.

In another patent application (EA013514B1) a method for modification of endovascular device made from metals or metal alloys such as stainless steel, cobalt-chromium alloy, titanium Ti or an alloy thereof and Cr chromium alloy, where coating of a tubular body's surface with a thin layer(s) of inert and biocompatible titanium-based material is disclosed. Such material minimally interacts with the tissues of the vessel walls and blood flow and does not adversely affect the human body. The coating is carried out using magnetron sputtering to create a thin film of titanium or a titanium alloy on the tubular body's surface. The thickness of the coating can be controlled by adjusting the deposition parameters, such as the deposition rate and chamber pressure. The resulting coating is comprised of titanium nitride ceramic coating.

Patent application EP3496776A1 discloses s method for producing desired morphology of a TiO2 nanotubular matrix, in particular titanium dioxide-containing matrix, which reduces adhesion and activation of platelets on medical devices by electrochemical anodisation. Surfaces produced by the abovementioned method can be used for blood-contacting devices, such as stents and artificial heart valves to reduce thrombus reactions on the implant material surface. The method of treatment is solely electrochemical anodisation with Pt electrode as a cathode and titanium-based substrate as an anode and does not involve any type of plasma.

Similarly, patent US20180228937A1 discloses the formation of biocompatible nanostructures with increased surface area, such as with nanotube and nanopore array on the surface of metallic, ceramic, or polymer materials for enhanced cell and bone growth, for in vitro and in vivo testing, cleansing reaction, implants and therapeutics. The method provides nanostructured substrates coated with Ti, TiO₂ or related metal and metal oxide films, which can be prepared by a method comprising one or more processes selected from DC or RF sputter deposition, oblique incident evaporation, chemical vapour deposition, laser surface melting and solidification, RF surface melting and solidification, chemical etching, patterned-mask-guided chemical, reactive ion etching and a combination thereof. However, the method of US20180228937A1 does not involve electrochemcial anodisation and atmospheric plasma as described herein.

In a patent application US20110159273A, the method for coating surfaces (medical devices including implants) with micro- and nanoparticles (by chemical bonds) with the aid of non-thermal low-pressure plasma method where O₂, Ar etc. are used as a carrier gas are disclosed. Plasma is used for fixation of the micro and nanoparticles (such as silver nanoparticles, titanium dioxide nanoparticles, diamond coatings, hydroxyapatite nanoparticles, metal/ceramic coatings and ceramics, organic nanofibers and composite materials and nanostructured aluminium oxide surfaces, etc.) on the surface of medical devices. By the method above adhesion of microorganisms to the surface of medical products or instruments is reduced. The method of treatment involves non-thermal low-pressure plasma treatment.

A method for surface treatment of a substrate is described in US006006763A where a gas discharge at or about atmospheric pressure produces activated gas or activated species which are further used for surface modification. Metal oxide films can be prepared by a gas discharge at or about atmospheric pressure that produces activated gas or active species. This method oxidizes or reduces the surface of a work that is processed, removing or cleaning organic or inorganic substances from the surface, mainly for its use as semiconductor devices. In this application atmospheric plasma in combination with liquid is used for the removal of substances.

Moreover, the method of treatment is different, as by our method of the invention the modified surface actually acts as a second electrode and thus the modification is not solely done by active species created in liquid but also by electrochemical anodization combined with atmospheric pressure plasma. In SHARMA R ET AL (INDUSTRY APPLICATIONS SOCIETY ANNUAL MEETING, 2008. IAS '08. IEEE, IEEE, PISCATAWAY, NJ, USA, 5 October 2008 (2008-10-05), ISBN: 978-1-4244-2278-4), it is disclosed surface modification of Ti substrates. The step of electrochemical anodization leads to the formation of nano structures, thereafter, the substrate was subjected to a plasma treatment at atmospheric pressure (see examples and claims). No mention of the simultaneous application of the plasma to the electrochemical step, nor of the post UV treatment.

### Object of the present invention

Therefore, an object of the present invention is to provide a method according to the claims, to obtain improved biocompatibility of medical devices, especially implantable medical devices.

### Summary of the present invention

The object of the present invention is solved according to the feature combination of claim 1. According to the present invention a method for treatment of a medical device or a blood connecting device made from Ti or Ti alloy, such as stent or the like, is disclosed. A blood-connecting device may be an implantable device that can be used for a human or animal host. These devices may come in direct contact with the blood of a patient but also with other devices like orthopedic or maxillofacial devices.

According to the invention, a metal medical device is provided. Afterwards immersing of the medical device in a liquid and depositing of a micro/nanostructured titanium oxide layer on the surface of the metal medical device by means of an electrochemical anodisation, whereby an electrode generates atmospheric pressure plasma, to allow creating active species in the liquid is performed. Finally treating the metal medical device by ultraviolet radiation (UV), wherein the UV source preferably emits a wavelength of 365 nm or below with an aerial power density of 3 to 6 mW cm⁻², and especially 5 mW cm⁻² is performed as defined in the claims.

According to the present invention modification of medical metallic surfaces comprising titanium and its alloys can be achieved by using a novel methodology that combines atmospheric pressure plasma and electrochemical anodisation method without the need to use environment unfriendly chemicals. This enables the fabrication of nanoscale morphology on the surface consisting of superior chemical properties which improve its biological response.

This specifically modified surface according to the invention significantly reduces bacterial adhesion, as well as prevents platelet adhesion and activation. Such surfaces are relevant for medical devices in contact with blood as they significantly reduce the incidence of life-threatening clinical events, such as thrombosis. The method of the invention improves the antibacterial properties of the surface of Ti and Ti alloys, which is relevant for all materials where bacterial infections should be reduced, especially in the case of medical devices, medical tools or equipment where bacterial infections and/or biofilm formation presents a serious concern.

The conventional electrochemical anodisation method uses typically platinum as a cathode and strong acids and other corrosive materials such as hydrogen fluoride or ammonium fluoride as the electrolyte. According to the methodology of the present invention, an atmospheric pressure plasma jet is used as a cathode and additionally, environment-friendly liquids are used to achieve micro and nanotopography of the surface with superior chemical properties. Through plasma-induced anodisation a thin film of micro and nano-structured oxide layer is formed on the surface which improves wettability and biocompatibility of the material. Ageing of the medical devices being used in orthopedic and cardiovascular applications present a serious challenge. Therefore, the present methodology also discusses the effect of ultraviolet regeneration/ activation and ageing of the samples prepared by the herein-disclosed method of the invention.

The micro and nanostructured oxide surface fabricated by the novel methodology inhibits activation and adhesion of platelets on the surface exhibiting improved hemocompatibility. This in turn reduces the risk of thrombosis. In addition, the modified surface also shows improved antibacterial efficacy towards a common pathogen i.e. E. coli., which is essential to improve the efficiency and longevity of medical devices. Moreover, similar results were obtained for UV regenerated/ activated and aged samples prepared by the method of the invention.

Thus, the insufficient biocompatibility of metal medical implants/devices, especially blood-connecting devices, consisting of titanium (Ti) and titanium alloys is solved. Although various surface modifications of metals and alloys were employed, the implantation of metal-based blood-connecting implants/devices still presents a risk of surface-induced thrombosis (formation of a blood clot due to platelet adhesion, aggregation and activation on such surfaces) and restenosis (narrowing of the blood vessel due to extensive smooth muscle cells proliferation). These clinical conditions (thrombosis, restenosis) can cause the failure of cardiovascular devices, which can lead to the removal of devices. In addition, an obstacle to overcome is also the danger of bacterial infections which affect the efficiency and longevity of all implantable devices as well as medical tools and other medical materials used in hospitals. Different approaches have been proposed to overcome these issues, like decorating nanostructures with antimicrobial nanoparticles or functionalization the nanostructures with anti-inflammatory agents and loading drugs inside the nanostructures. However, the success rate of such approaches is limited mainly due to expensive and complicated procedures for fabrication and issues with the toxicity of antibacterial coatings.

Thus, by the novel method of the invention presented herein, micro and nanostructured titanium oxide surface is fabricated, which reduces platelet adhesion and activation. Nano-topographic features with specific surface chemistry influence on the cell-surface interactions such as platelet adhesion and activation as well as bacterial adhesion.

### Brief description of the drawings

The invention is herein described, by way of example, with reference to the accompanying table/drawings, wherein:
Table 1 shows the chemical composition of the surface of materials examined by X-ray photoelectron spectroscopy, wherein the differences between untreated Ti substrate and Ti substrate treated by the method of the invention are presented (accordingly after treatment according to the method of invention a reduction in Ti/O ratio is observed);
Figure 1 presents surface morphology of Ti substrate after applying the method of the invention, as imaged by SEM, whereby the SEM analysis revealed that the morphology of the plasma anodised surfaces is micro/nanostructured; consisting of flower-like morphology on the surface.
Figure 2 shows SEM image of untreated Ti substrate after incubation with whole blood (magnification 3000x), thereby platelets observed on the surface attain dendritic, spread and fully spread form, which is correlated with high platelet activation on the surface that indicates high risk for thrombosis;
Figure 3 shows a SEM image of a Ti substrate treated by method of invention, after incubation with whole blood (magnification 3000x), thereby only some platelets can be observed on the surface, mainly in non-activated form (round or dendritic), which reduces the risk of thrombosis;
Figure 4 shows antibacterial effects of samples against *E. coli* growth on the surface; and
Figure 5 schematically depicts a system suitable to perform the inventive methodology, whereby the medical device is positioned according to two different positions (Fig. 5A and Fig. 5B).

### Detailed description of the invention

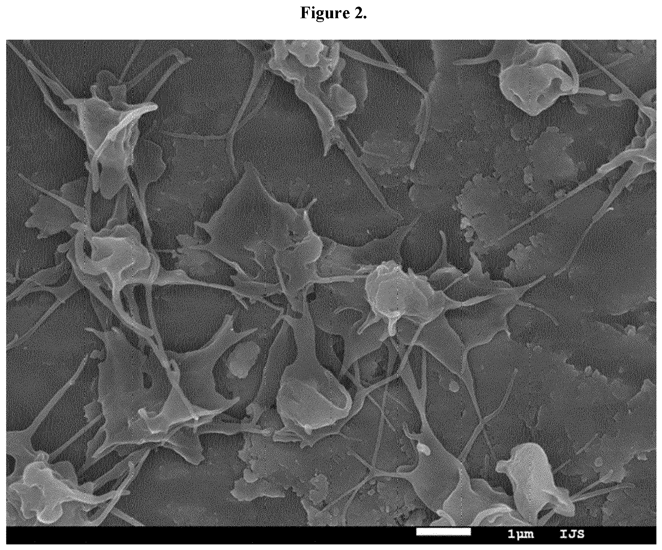

Against state-of-the-art, the method of the invention enables the formation of a stable film of titanium oxide consisting of micro/nano flower-like morphology on the surface. The morphology of the surface after plasma-induced anodisation was examined by scanning electron microscopy (SEM) as shown in the figures 2 to 4.

From the SEM images, it can be seen that the morphology of the surface is just like clusters protruding from the surface. It could be established that these clusters aggregate to form the petals of flower-like microstructures seen on the surface. The biocompatibility of the modified Ti substrate treated by the method of the invention is confirmed by the *in vitro* biological studies, where interaction with platelets was studied. Furthermore, the antibacterial effect of a modified Ti substrate covered by the oxide layer was studied to examine the effect of micro/nanoflowers on bacterial cell attachment. Biocompatibility of materials treated according to the procedure improved significantly since the platelet adhesion was minimized. Moreover, the methods of the invention inhibit bacterial growth on the surface providing an antibacterial effect against *E*. *coli* which was further reduced upon UV regeneration/ activation. For a one-year-old (aged) sample prepared by the method of invention, similar results were obtained.

The innovative technique involves two relevant steps: first is use of electrochemical anodization combined with atmospheric plasma in liquids and second is UV treatment, which presents the final step and improves biological response. The disclosed technique enables the formation of a high-quality micro and nano-structured titanium oxide layer on the surface of the said metallic substrate, which significantly influences the biological response.

Titanium and titanium alloys treated with the innovative method presented herein exhibit antibacterial properties and are hemo-compatible. Platelet adhesion and activation on such surfaces is significantly reduced. This lowers the risk of thrombosis events after implantation. Bacterial adhesion on surfaces treated by the method of the invention is also prevented.

The method of the invention addresses the need for improved biocompatibility of materials made from metal alloys, in particular vascular stents, with no exclusion to other medical devices, such as orthopaedic or maxillofacial implants. The present invention involves;

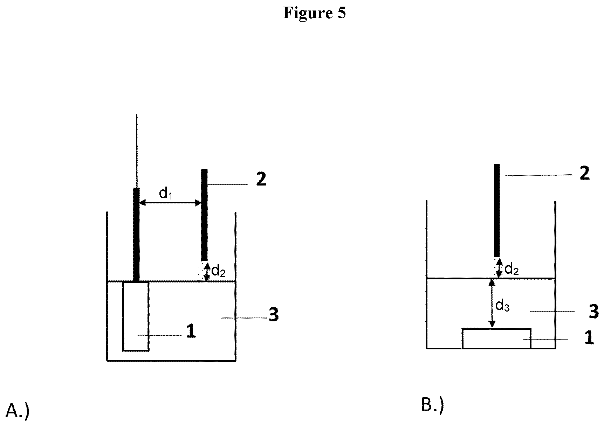

Step 1: Electrochemical anodisation with atmospheric pressure plasma which enables formation of active species in the environment-friendly liquid (electrolyte); deposition of titanium dioxide film on the surface of said substrate by by means of an electrochemical anodisation with an additional electrode generating atmospheric pressure plasma enables the formation of titanium oxide layer, consisting of micro and nano-flowers. That is an electrode is used to generate atmospheric plasma. The position of sample during treatment may vary, the two possible examples are presented in Figures 5A and 5B.

Step 2: UV irradiation, herein samples treated with step 1 are irradiated with UV-A to regenerate/activate the surface.

Against state-of-the-art, the method of the invention enables the formation of a stable film of titanium oxide consisting of micro and nanoflowers. The first step assures the appropriate size and geometry of the features within the stable film of titanium oxide. In the second step, UV treatment enables additional activation of the surface to optimise biological response also on aged surfaces.

The morphology of the surface after performing the two-step treatment was examined by scanning electron microscopy (SEM). The hemocompatibility of the materials made from Ti substrate treated by the methods of the invention is confirmed by the interaction with platelets. The interactions between Ti substrate and platelets were monitored by SEM. Biocompatibility of materials treated according to the two-step procedure improved significantly since the platelet adhesion was minimized.

The present invention comprises two steps: Step 1.) electrochemical anodisation with atmospheric pressure plasma as an electrode and Step 2.) UV-A irradiation. In the first step, the metal surface of titanium or titanium alloy is exposed to liquid, where an atmospheric pressure plasma jet is used to activate the liquid and at the same time act as a counterelectrode i.e. cathode.

This atmospheric pressure plasma jet setup consists of tubular capillary (discharge microreactor) made of an insulating material, a couple of electrodes, radio frequency (RF) step-up transformer, RF power supply, thin long metallic tube placed inside the glass tube as a powered electrode and gas supply system in order to maintain the desired flow rate of gas, primarily of Ar or other noble gas or their combination with air, oxygen or nitrogen. The treated material; titanium or titanium alloy is used as a second electrode. Through this method a layer of oxide composed of micro and nano-structured titanium oxide is obtained on the surface. The surface consists of micro-flower-like morphology of titanium oxide layer. The process may be performed at room temperature or any convenient temperature which prevents thermal damage to the substrate. The surface of the metal is exposed to activated species from plasma and is used as an anode, which allows the surface to be oxidized and micro/nanostructured.

In the second step, the obtained micro/nano-structured titanium oxide surface is exposed to ultraviolet radiation from a UV lamp (UV-A intensity of 2000 µW/cm²). These surface features along with UV sterilisation/activation significantly influence the biological response, as platelet and bacterial adhesion is significantly reduced. This is of high importance for medical devices in contact with blood (like vascular stents).

The efficacy of the Ti substrates has been established in the present invention through *in vitro* approaches:
a.) evaluation of hemocompatibility by incubation with whole human blood control Ti sample, a sample after performing the Steps 1 and 2 separately, and a sample treated by the innovative two-step method.
b.) evaluation of antibacterial efficiency of the control Ti sample, a sample after performing steps 1 and 2 separately, and a sample treated by the innovative two-step method.

The efficiency of the methods of the invention will be demonstrated in the following examples, in which Ti material was used as a substrate. The application of the method of the invention is not limited to Ti, as also Ti based materials can be applied, such as nickel-titanium/Nitinol (NiTi) and Ti₆Al₄V.

### Example 1: Ti substrate- platelet adhesion and activation and antibacterial effect

In the example disclosed herein control Ti substrate was analysed by XPS to obtain information about the chemical composition of the surface. The untreated Ti substrate chemical composition is presented in Table 1, Ti substrate consists of about 41.2 at. % of oxygen, 38.3 at. % of carbon and 18 at. % of titanium, where the Ti/O ratio is 0.43

The adhesion and activation of platelets on the control Ti substrate was performed according to the following procedure: Ti substrates were cleaned with ethanol and dried under a nitrogen stream. Whole blood incubation: The blood was taken by vein puncture from a healthy human donor. The blood was drawn into 3 ml tubes with trisodium citrate anticoagulant (Sigma Aldrich). Afterwards, the fresh blood (300 µl) was incubated with Ti substrates in 24 well plates for 30 min at room temperature.

After incubation, the blood was removed, and 250 µl of phosphate-buffered saline (PBS) was added to the Ti substrates. Ti substrates were rinsed 3 times with 250 µl PBS in order to remove weakly adherent platelets. Adherent biological material was subsequently fixed with 250 µl of 1 % GA (glutaraldehyde) solution with the addition of 250 µl PBS for 1h at room temperature.

Afterwards, the surfaces were rinsed with 250 µl PBS and then dehydrated using a graded ethanol series (50, 70, 80, 90, 100 and again 100 vol. % ethanol) for 5 min and in the last stage in the series (100 vol.% ethanol) for 15 min. Afterwards, the samples were placed in a container with liquid nitrogen and further kept in vacuum overnight. This drying process preserves the natural structure of the sample and avoids surface tension which could be caused by normal drying. The dried samples were subsequently coated with gold/palladium and examined by means of SEM (Carl Zeiss Supra 35 VP). Evaluation of platelet density, adhesion and activation were deduced from SEM images. Results obtained by SEM analysis clearly indicate that platelets readily attach on the surface of Ti substrate with lamellipodia and filopodia and start to aggregate, as shown in Figure 2. Observed morphology of platelets can be described as fully spread. Such morphology of platelets has a high potential to cause thrombosis.

Evaluation of platelet density, adhesion and activation were deduced from SEM images. Results obtained by SEM analysis clearly indicate that platelets readily attach on the surface of Ti substrate with lamellipodia and filopodia and start to aggregate, as shown in Figure 2. Observed morphology of platelets can be described as dendritic and spread. Such morphology of platelets has a high potential to cause thrombosis.

Antibacterial test on Ti substrate (control) was performed according to ISO22196 protocol for evaluation of the antibacterial effect. The pathogenic strain of *Escherichia coli* (*E. coli*) was first prepared in Luria-Bertani broth for 24 hours at 370C. A suspension of E. coli (105 colony forming unit (CFU)/mL) was prepared, from which 0.1 mL was pipetted onto the surface of Ti substrate. The samples were then incubated in the incubator (I-105 CK UV, Kambič) for 24 hours at 37°C in a humidity box in order to maintain relative humidity at 90%. After incubation, E. coli on the surface was removed using 2.5 mL of sterilised phosphate buffered saline (PBS) and 0.2 mL of this solution was taken for inoculation of E. coli in the Nutrient agar plate at 37°C for 24 hours. Then the number of CFUs can be determined. For convenient counting of CFUs, before inoculating E. coli in the Nutrient agar plate, the initial solution was diluted further with PBS by a factor of 100-105. The CFU/mL were calculated using an automated colony counter (Acolyte 3, Synbiosis).

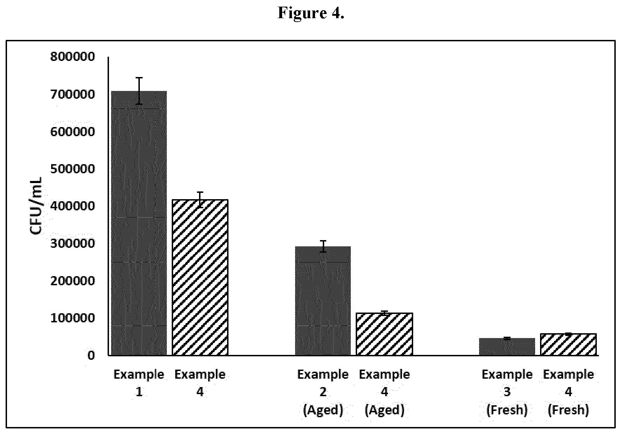

Through antibacterial assay (Figure 4), it was determined that on untreated Ti substrate significant bacterial growth (approximately 708166 CFU/mL) of E. coli was observed.

### Example 2: Ti substrate treated with the first step of the method of invention (fresh sample)-platelet adhesion and activation and antibacterial effect i.e. Step 1.

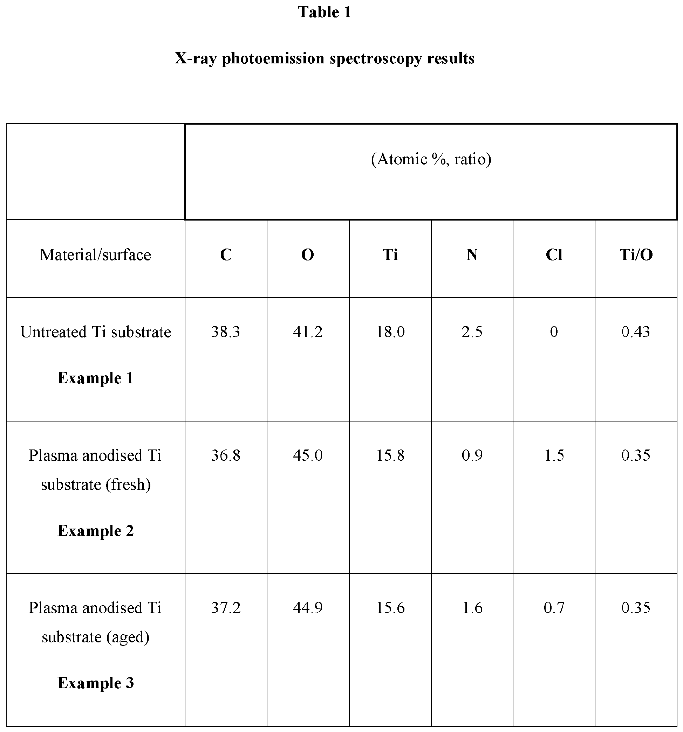

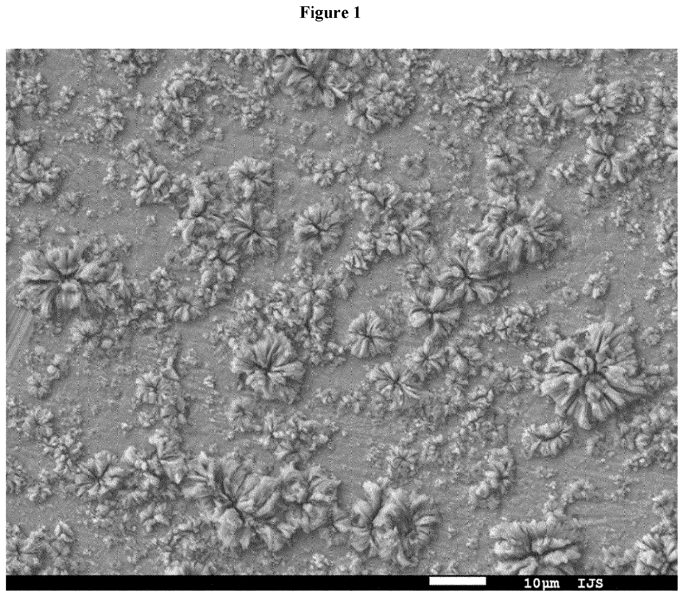

Ti substrate was put into the electrolyte cell filled with the ethylene glycol-based electrolyte solution and sodium chloride. Ti substrate was anodised at 50V for 30 minutes with atmospheric pressure plasma being operated at 1.4A and 4.6V with flow rate of 1.0 sccm for helium (step 1). After the anodisation, samples were thoroughly washed and kept in deionised water for a few days and later dried in a conventional furnace at 700 C for 1 hour. Surface morphology is presented in Figure 1, while compositional analysis obtained from XPS is presented in Table 1. The resulting surface consists of micro/nano-flower like morphology and from the inset in Figure 1, it can be concluded that the nanowires cluster together to form flower-like morphology on a microscale. From inset, it can be seen that the nanowires bundle together to form petals of this flower like morphology according to the invention.

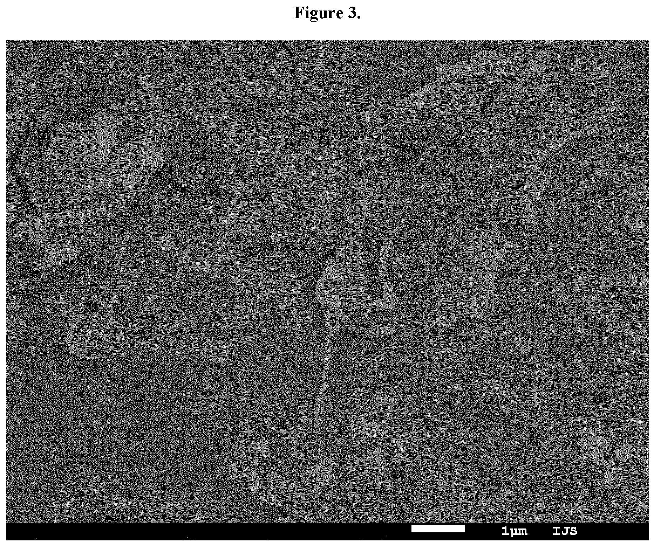

The incubation procedure with whole blood was the same as the one described in Example 1, the substrate was used no longer than 3 days after fabrication (fresh sample). Results of interaction between platelet and plasma anodised Ti substrate (Step 1) can be observed from Figure 3. It clearly indicates that almost no platelets are detected on the surface, while those attached to the surface are not as extensively spread as in example 1. Hence, the surfaces treated with this method would relatively decrease the probability of thrombosis and related events.

The fabricated micro/nano-structured morphology and specific surface chemistry influence platelet adhesion. Significantly lower platelet interaction compared to untreated Ti substrate presented in Figure 2 is observed. There is hardly any platelet detected on the surface and those platelets that are detected are not aggregating and are in a more round (not activated) form, which lowers the risk of thrombosis.

The antibacterial test was the same as the one described in Example 1, the substrate was used no longer than 3 days after fabrication (fresh sample). The Ti substrate treated with plasma anodisation (step 1) stimulates the antibacterial effect against E. *coli* as it shows 93% reduction in bacterial growth on being compared to untreated Ti sample.

### Example 3: Ti substrate treated with method of invention (aged sample)- antibacterial effect i.e. Step 1.

The dried samples prepared according to Example 2 were sealed and kept inside air tight boxes for 1 year to age the samples. Even after the aging, no significant change in morphology was observed for the sample. However, minimal changes were observed in the chemical composition of aged samples as compared to fresh ones. After ageing about 45 at. % of oxygen, 36.8 at. % of carbon and 15.8 at. % of titanium was present on the surface, while the Ti/O ratio was 0.35.

The antibacterial test was the same as the one described in Example 1, while the substrate was used at least 6 to 12 months after fabrication (aged sample). Even after being aged the samples depicted efficacy against *E*. *coli* as 59% reduction in bacterial growth was observed in comparison to sample in Example 1 i.e. untreated.

### Example 4: Ti substrate treated with Step 1 and Step 2- antibacterial effect.

The substrates from Example 1, 2 and 3 were additionally treated with UV so that its effect can be studied on these surfaces. The adhesion and activation of platelets and antibacterial test were done as described in Example 1. After treatment of samples with UV lamp (UV-A intensity of 2000 µW/cm2) at a distance of 3 cm for 10 minutes the untreated Ti substrate (Example 1), exhibited 40% reduction in bacterial growth compared to untreated Ti substrate (Figure 4). After UV treatment of the substrate from Example 2 similar results were observed as 86% reduction was observed compared to previous substrate. Similarly for aged substrate (Example 3) upon UV treatment, antibacterial effect was still maintained with 73% reduction w.r.t UV sterilised untreated Ti substrate. This shows that even aged substrate can be rejuvenated after UV treatment, as reactive oxygen species can be released upon UV irradiation that would assist in rupturing bacterial cell membrane. The studies for platelet adhesion and activation were the same as presented in Example 1.

For untreated Ti substrate approximately 42% reduction in bacterial growth was observed after UV treatment. In the case the samples treated with the method of invention Example 2 the reduction in bacterial growth was significantly higher and was reported to be 93%. A similar effect was reported for aged samples Example 3 i.e. 73%.

Figure 5A and 5B schematically shows a system suitable to process the method according to the invention.

According to the embodiment of Figure 5A the medical device/substrate 1 is positioned horizontally distanced to the atmospheric pressure plasma. The medical device 1 may essentially correspond to a tube shape defining an axis which is generally parallel to the axis of the additional electrode or electrode providing/generating atmospheric plasma, respectively, as can be seen in Fig. 5A. However, the medical device can be any other shape-and is not limited to the aforementioned tubular shape, thusrelevant is the maximum distance from the axis of medical device and additional electrode (that is the electrode generating atmospheric pressure plasma).

In contrast to Figure 5B shows that the medical device/substrate is positioned vertically disposed to the atmospheric pressure plasma device 2 according to a second embodiment.

As can be seen with reference to Fig. 5B the overall distance between plasma source 2 and medical device 1 is d₂ + d₃ which in turn corresponds to the distance d₁ of Fig. 5A

In Fig. 5A the horizontal distance between the medical device/substrate 1 and the atmospheric pressure plasma 2 is between 0.5 to 6 cm, preferably between 1 to 3.5 cm, while the distance between the atmospheric pressure plasma and the liquid 3 surface is between 1 to 40 mm, preferably 4 to 10 mm.

It was surprisingly observed that these values lead to an optimal surface of the coated medical device according to the present invention.

Therefore, according to the present invention a micro and nanostructured titanium oxide surface for medical devices is provided, which reduces platelet adhesion and activation. Nano-topographic features with specific surface chemistry positively influence on the cell-surface interactions such as platelet adhesion and activation as well as bacterial adhesion.

## Claims

1. A method of coating a metal medical device (1), especially vascular stent, comprising:
• providing a metal medical device (1);
• immersing the medical device (1) in a liquid (3) and depositing of a micro/nanostructured titanium oxide layer on the surface of the metal medical device by means of an electrochemical anodisation, whereby an electrode (2) generates atmospheric pressure plasma, to allow creating active species in the liquid (3);
• treating the metal medical device (1) by ultraviolet radiation (UV), wherein the UV source preferably emits a wavelength between 200 and 400 nm, preferably with a wavelength of 365 nm, and with an aerial power density of 3 to 6 mW cm⁻², and especially 5 mW cm⁻²,
• wherein the liquid (3) comprises solely deionised water and/or additional salts like NaCl, KCI, PBS and/or ethylenglycol, NaOH, glucose and/or natural extracts and combinations thereof with a pH value preferably in a range between 7 to 13 and a temperature value preferably in a range between 0 and 50 C°, preferably between 15 and 25 C°, and
• wherein the metal medical device (1) comprises titanium, titanium alloys and/or nickel-titan/ Nitinol (Ni-Ti).

2. Method according to at least one of the preceding claims, wherein the electrode (2) generating atmospheric pressure plasma is a pressure plasma jet setup, comprising a tubular capillary discharge microreactor, a couple of electrodes, a radio frequency (RF) step-up transformer, a RF power supply, a thin metallic tube disposed inside a glass tube as a powered electrode and gas supply system.

3. Method according to at least one of the preceding claims, wherein the gas for generation of the atmospheric pressure plasma is Ar or He with or without addition of oxygen, nitrogen or air or a combination thereof.

4. Method according to at least one of the preceding claims, wherein the immersing of the medical device in the liquid (3) provides coating of the surface with the nano-structured titanium oxide layer consisting of a micro and/or nano-flower like topography.

5. Method according to at least one of the preceding claims, wherein the horizontal distance (d₁) between an axis of the medical device (1) and an axis of the electrode (2) generating atmospheric pressure plasma is between 0.5 to 6 cm, preferably between 1 to 3.5 cm, while the distance between the electrode (2) and the liquid surface is between 1 to 40 mm, preferably 4 to 10 mm.

6. Method according to at least one of the preceding claims, where treating with UV radiation is performed between 5 to 90 min, preferably between 5 and 20 min.

7. Method according to at least one of the preceding claims, wherein the atmospheric pressure plasma provides active species in liquid, which enable electrochemical anodization.

8. Method according to one of the preceding claims 1 to 4, wherein the medical device (1) is vertically disposed within the fluid (3) and the distance between axis of medical device and the fluid surface (d3) is between 0.5 to 6 cm, preferably between 1 to 3.5 cm, whereby an axis of the plasma source (2) is essentially perpendicular to an axis of the medical device (1).

## Patentansprüche

1. Verfahren zum Beschichten einer medizinischen Metallvorrichtung (1), insbesondere eines Gefäßstents, umfassend:
• Bereitstellen einer medizinischen Metallvorrichtung (1);
• Eintauchen der medizinischen Vorrichtung (1) in eine Flüssigkeit (3) und Abscheiden einer mikro/nanostrukturierten Titanoxidschicht auf der Oberfläche der medizinischen Metallvorrichtung mittels elektrochemischer Anodisierung, wobei eine Elektrode (2) ein Atmosphärendruckplasma erzeugt, um die Bildung aktiver Spezies in der Flüssigkeit (3) zu ermöglichen;
• Behandeln der medizinischen Metallvorrichtung (1) mit ultravioletter Strahlung (UV), wobei die UV-Quelle vorzugsweise eine Wellenlänge zwischen 200 und 400 nm, vorzugsweise eine Wellenlänge von 365 nm, und eine Flächenleistungsdichte von 3 bis 6 mW cm⁻², insbesondere 5 mW cm⁻², emittiert,
• wobei die Flüssigkeit (3) ausschließlich deionisiertes Wasser und/oder zusätzliche Salze wie NaCl, KCl, PBS und/oder Ethylenglykol, NaOH, Glukose und/oder natürliche Extrakte sowie Kombinationen davon umfasst, mit einem pH-Wert vorzugsweise im Bereich zwischen 7 und 13 und einer Temperatur vorzugsweise im Bereich zwischen 0 und 50 °C, vorzugsweise zwischen 15 und 25 °C, und
• wobei die medizinische Metallvorrichtung (1) Titan, Titanlegierungen und/oder Nickel-Titan/Nitinol (Ni-Ti) umfasst.

2. Verfahren nach wenigstens einem der vorstehenden Ansprüche, wobei die Atmosphärendruckplasma erzeugende Elektrode (2) eine Druckplasma-Jet-Anordnung ist, die einen röhrenförmigen Kapillarentladungs-Mikroreaktor, ein Elektrodenpaar, einen Hochfrequenz- (HF)-Aufwärtstransformator, eine HF-Stromversorgung, ein dünnes Metallrohr, das als stromführende Elektrode in einem Glasrohr angeordnet ist, sowie ein Gasversorgungssystem umfasst.

3. Verfahren nach wenigstens einem der vorstehenden Ansprüche, wobei das Gas zur Erzeugung des Atmosphärendruckplasmas Ar oder He mit oder ohne Zugabe von Sauerstoff, Stickstoff oder Luft oder einer Kombination davon ist.

4. Verfahren nach wenigstens einem der vorstehenden Ansprüche, wobei das Eintauchen der medizinischen Vorrichtung in die Flüssigkeit (3) eine Beschichtung der Oberfläche mit der nanostrukturierten Titanoxidschicht bewirkt, die aus einer mikro- und/oder nano-blumenartigen Topographie besteht.

5. Verfahren nach wenigstens einem der vorstehenden Ansprüche, wobei der horizontale Abstand (d₁) zwischen einer Achse der medizinischen Vorrichtung (1) und einer Achse der das Atmosphärendruckplasma erzeugenden Elektrode (2) zwischen 0,5 und 6 cm, vorzugsweise zwischen 1 und 3,5 cm, beträgt, während der Abstand zwischen der Elektrode (2) und der Flüssigkeitsoberfläche zwischen 1 und 40 mm, vorzugsweise zwischen 4 und 10 mm, beträgt.

6. Verfahren nach wenigstens einem der vorstehenden Ansprüche, wobei die Behandlung mit UV-Strahlung zwischen 5 und 90 min, vorzugsweise zwischen 5 und 20 min, durchgeführt wird.

7. Verfahren nach wenigstens einem der vorstehenden Ansprüche, wobei das Atmosphärendruckplasma aktive Spezies in der Flüssigkeit bereitstellt, die eine elektrochemische Anodisierung ermöglichen.

8. Verfahren nach einem der vorstehenden Ansprüche 1 bis 4, wobei die medizinische Vorrichtung (1) vertikal in der Flüssigkeit (3) angeordnet ist und der Abstand zwischen der Achse der medizinischen Vorrichtung und der Flüssigkeitsoberfläche (d₃) zwischen 0,5 und 6 cm, vorzugsweise zwischen 1 und 3,5 cm, beträgt, wobei eine Achse der Plasmaquelle (2) im Wesentlichen senkrecht zu einer Achse der medizinischen Vorrichtung (1) steht.

## Revendications

1. Procédé de revêtement d'un dispositif médical métallique (1), notamment d'un stent vasculaire, comprenant :
• fournir un dispositif médical métallique (1);
• immerger le dispositif médical (1) dans un liquide (3) et déposer une couche d'oxyde de titane micro/nanostructurée sur la surface du dispositif médical métallique au moyen d'une anodisation électrochimique, une électrode (2) générant un plasma à pression atmosphérique afin de permettre la création d'espèces actives dans le liquide (3);
• traiter le dispositif médical métallique (1) par rayonnement ultraviolet (UV), la source UV émettant de préférence à une longueur d'onde comprise entre 200 et 400 nm, de préférence à une longueur d'onde de 365 nm, et avec une densité de puissance surfacique de 3 à 6 mW cm⁻², et notamment de 5 mW cm⁻²,
• dans lequel le liquide (3) comprend uniquement de l'eau déionisée et/ou des sels additionnels tels que NaCl, KCl, PBS et/ou de l'éthylène glycol, du NaOH, du glucose et/ou des extraits naturels et des combinaisons de ceux-ci, avec une valeur de pH de préférence comprise dans une plage allant de 7 à 13 et une valeur de température de préférence comprise dans une plage allant de 0 à 50 °C, de préférence entre 15 et 25 °C, et
• dans lequel le dispositif médical métallique (1) comprend du titane, des alliages de titane et/ou du nickel-titane / Nitinol (Ni-Ti).

2. Procédé selon au moins l'une des revendications précédentes, dans lequel l'électrode (2) générant le plasma à pression atmosphérique est un dispositif à jet de plasma sous pression, comprenant un microréacteur tubulaire à décharge capillaire, une paire d'électrodes, un transformateur élévateur à radiofréquence (RF), une alimentation RF, un tube métallique mince disposé à l'intérieur d'un tube en verre en tant qu'électrode alimentée, et un système d'alimentation en gaz.

3. Procédé selon au moins l'une des revendications précédentes, dans lequel le gaz destiné à la génération du plasma à pression atmosphérique est de l'Ar ou de l'He avec ou sans ajout d'oxygène, d'azote ou d'air, ou d'une combinaison de ceux-ci.

4. Procédé selon au moins l'une des revendications précédentes, dans lequel l'immersion du dispositif médical dans le liquide (3) assure le revêtement de la surface avec la couche d'oxyde de titane nanostructurée consistant en une topographie de type micro-fleur et/ou nano-fleur.

5. Procédé selon au moins l'une des revendications précédentes, dans lequel la distance horizontale (d₁) entre un axe du dispositif médical (1) et un axe de l'électrode (2) générant le plasma à pression atmosphérique est comprise entre 0,5 et 6 cm, de préférence entre 1 et 3,5 cm, tandis que la distance entre l'électrode (2) et la surface du liquide est comprise entre 1 et 40 mm, de préférence entre 4 et 10 mm.

6. Procédé selon au moins l'une des revendications précédentes, dans lequel le traitement par rayonnement UV est effectué pendant 5 à 90 min, de préférence pendant 5 à 20 min.

7. Procédé selon au moins l'une des revendications précédentes, dans lequel le plasma à pression atmosphérique fournit des espèces actives dans le liquide, lesquelles permettent l'anodisation électrochimique.

8. Procédé selon l'une des revendications précédentes 1 à 4, dans lequel le dispositif médical (1) est disposé verticalement dans le fluide (3) et la distance entre l'axe du dispositif médical et la surface du fluide (d₃) est comprise entre 0,5 et 6 cm, de préférence entre 1 et 3,5 cm, un axe de la source de plasma (2) étant essentiellement perpendiculaire à un axe du dispositif médical (1).
